# EUROPEAN PATENT SPECIFICATION

(11) **EP 4 580 655 B1**
(45) Date of publication and mention of the grant of the patent: **09.09.2026**
(21) Application number: 24751213.0
(22) Date of filing: 23.07.2024
(51) Int. Cl.: A61K 38/47, A61K 35/76, A61K 9/19, A61K 47/26, A61P 31/04

(54) **AN ANTIMICROBIAL MIXTURE COMPRISING BACTERIOPHAGES AND ENDOLYSIN**
EINE ANTIMIKROBIELLE MISCHUNG AUS BAKTERIOPHAGEN UND ENDOLYSIN
UN MÉLANGE ANTIMICROBIEN COMPRENANT DES BACTÉRIOPHAGES ET DE L'ENDOLYSINE

(30) Priority: 31.07.2023 CZ 20230293
(43) Date of publication of application: 09.07.2025
(73) Proprietor: MB Pharma Group s.r.o., 12000 Praha 2 (CZ)
(72) Inventor: MOSA, Marek, 29477 Meceriz (CZ); BENESIK, Martin, 68765 Strani (CZ)
(74) Representative: Jirotkova, Ivana
(86) International application number: PCT/CZ2024/050050
(87) International publication number: WO 2025/026474

(56) References cited:
- WO-A2-01/51073
- US-B2- 10 676 721
- LU HAN ET AL: "Synergistic action of phages and lytic proteins with antibiotics: a combination strategy to target bacteria and biofilms", BMC MICROBIOLOGY, vol. 23, no. 1, 23 May 2023 (2023-05-23), GB, XP093216174, ISSN: 1471-2180, Retrieved from the Internet <URL:https://link.springer.com/article/10.1186/s12866-023-02881-2/fulltext.html> DOI: 10.1186/s12866-023-02881-2
- CHANG RACHEL YOON KYUNG ET AL: "Novel antimicrobial agents for combating antibiotic-resistant bacteria", ADVANCED DRUG DELIVERY REVIEWS, ELSEVIER, AMSTERDAM , NL, vol. 187, 4 June 2022 (2022-06-04), XP087114808, ISSN: 0169-409X, [retrieved on 20220604], DOI: 10.1016/J.ADDR.2022.114378

## Description

### Technical Field

The invention relates to a mixture for the preparation of lyophilized single-dose forms (tablets) comprising as the active ingredient bacterial viruses (bacteriophages or phages for short), whether in the form of phage lysates or purificates in combination with their antimicrobially acting enzymes (endolysins). The make-up of this form provides a balanced composition of individual constituents, ensuring suitable technical parameters for the dosage/administration form while maintaining maximum stability of the active ingredients (phages and endolysins) and maintaining their long-term stability. Accordingly, this administration form can be applied to the treatment of bacterial infections caused by the pathogenic strains *Staphylococcus aureus* and *Pseudomonas aeruginosa* in clinical practice. In particular, these are two specifically acting phages against the bacteria S. *aureus* and *P. aeruginosa* and the endolysin LysMB10 with a unique amino acid sequence.

### Background Art

The World Health Organization has included resistance of pathogenic bacteria to antibiotics among the biggest threats to human health in the 21st century, leading to not only increased mortality but also to considerable loading of the health care system (URL 1). The most dangerous pathogens resistant to antibiotics include the bacteria S. *aureus* and *P. aeruginosa.* Representatives of S. *aureus* are causal agents of a number of serious infections, e.g. osteomyelitis, sepsis as well as infections of wounds and skin. Similarly, *P. aeruginosa* also causes a wide range of diseases, e.g. infections of wounds and pneumonia (especially dangerous in patients with cystic fibrosis). Both the above mentioned pathogens are opportunistic, i.e. endangering mainly immunocompromised subjects, often in hospital environment (Barer 2018). Resistance of these pathogens thus complicates the provision of health care, especially limiting the possibilities of antibiotic treatment of infections and leading to the necessity to search for alternatives such as phage therapy (Abedon et al. 2011).

Bacteriophages (phages for short) are viruses of bacteria causing bacterial lysis. The phage therapy uses this characteristic to treat bacterial infections. This therapeutic approach has been investigated since the first half of the 20th century, however, after the discovery and spread of antibiotics, it was neglected in the Western countries (the research has been partly continued in former Soviet Union countries, e.g. in Georgia). The phages have only remained the focus of interest in the fields of biotechnology and molecular biology (Abedon et al. 2011). The discovery of antibiotics has been the main reason for this situation. However, phage therapy offers a number of benefits e.g. a lower risk of side effects (compared to antibiotics), mainly due to a minimal influence on the patient's native microflora, and further, phages can adapt to the defence mechanisms of bacteria (Loc-Carrillo and Abedon 2011). The initial insufficient knowledge of phage biology has led to ambiguous results of the therapeutic application. With the development of modern methods of molecular biology, whole-genome sequencing, genomics and proteomics, we have got to now sufficiently understand phage biology and its interaction with the host bacteria. With the wide scope of this knowledge concerning bacteriophages as well as due to the increasing antibiotic resistance, the interest in phage therapy is globally rising (Abdelrahman 2021). Antibacterial application of the phage enzymes themselves (called endolysins), which can lyse the host cell, has witnessed a similar rise of interest. These enzymes are involved in the phage life cycle in the last stage, when they cleave peptidoglycan of the cell wall, thus killing the bacterial cell. With gram-positive bacteria such as S. *aureus,* endolysins can also be applied from outside of the bacterial cell (Abdelrahman 2021). In both these biotechnological treatment protocols for bacterial infections, it is important to achieve the longest possible stability of their antimicrobial action in time and uniform dosage at the same time. Both the above mentioned characteristics are significant for the practical application of phages and their endolysins (Abdelrahman 2021).

Each bacteriophage differs in the spectrum of bacteria it can successfully infect and subsequently lyse. Phages may be polyvalent with a wide spectrum of bacteria they act against, but on the other hand also highly specialized in a single pathogenic microorganism. This is similar to endolysins, but these generally act against more species of bacteria within a genus, but they may differ in their activity (Abdelrahman 2021). It is the combination of different phages (a cocktail) that may provide an extension of the spectrum of antibacterial activity. In addition, isolation of specific natural mutants may result in antimicrobial activity against otherwise resistant bacteria. The bacteriophages mentioned here refer to specific phages that have been isolated as strains of phages active against resistant bacteria. In this way, they extend the spectrum of microbial activity to both the bacteria resistant to antibiotics and to those insensitive to other phages.

The use of phages and endolysins in liquid form is common, but the preparation of administration/medicament/storage forms is a great challenge for current science. According to the available literature, phages have been processed into liquid, semisolid as well as solid forms (Jault et al., 2019, Brown et al., 2018, Khanal et al., 2021). The preparation of liquid forms is not time or money consuming but they are more prone to microbial contamination and have higher demands on the storage room, where it is a problem to maintain the stability of their antibacterial activity at higher temperatures(Malik et al. 2017). Moreover, unlike semisolid and solid medicament forms, liquid forms have limitations in certain dosage forms. The main advantage of semisolid forms is easier administration, but they suffer from the same complications as liquid forms. In both cases, preservatives and antioxidants can be used to maintain the microbiological quality, but they may have a negative impact on the antimicrobial effect and quality of the resulting product (Subils et al., 2012). Solid forms generally have a lower risk of microbial contamination such that there is no need to apply preservatives. Moreover, they ensure higher stability of the antimicrobial effect, more accurate dosing and lower requirements for storage conditions. The main disadvantages comprise more complex optimization and higher financial demands of their preparation (Komárek 2006). Proven methods of processing phages into a solid form include spray drying and lyophilization (Malik et al. 2017).

During spray drying, a solution of the solid substance is exposed to hot and dry gas in a drying chamber, which results in the formation of powder particles that are evacuated from the drying chamber. Such a process is mainly applied in the manufacturing of inhalable powders (Hoe et al. 2014). In the case of phages, this method has been tested several times and good stability of the phage titre was achieved (Malik et al. 2017). However, as mentioned above, spray drying only produces powder particles that have a more complicated dosage and storage and require further processing, e.g. compression, which may negatively impact efficiency of the phages (Khanal et al., 2021) mainly due to temperature variations.

Lyophilization (freeze drying) is standardly used to process bacteriophages into solid form. This process comprises freezing the sample, followed by primary drying involving sublimation of ice, and finally, during secondary drying, the residual water is evaporated at higher temperatures (Malik et al. 2017). The phages in the lyophilizate can maintain efficiency for several months, even years (Clark 1962). However, this stability is influenced by many factors such as conditions of the lyophilization process, excipients used (i.e. mainly fillers and cryoprotectants), or storage and dissolution of the resulting lyophilizate (Malik et al. 2017). In addition, in contrast to chemicals, the stability and efficiency of a phage differs for each strain, therefore it is necessary to experimentally optimize the lyophilization process and to find the best composition of the media suitable to save the maximum activity of the particular phage. A similar principle works for enzymes as well.

What needs to be emphasized is that phages are standardly lyophilized in closed vials into a so-called lyophilization cake. This, however, is more complicated for administration and dosage; for example, each cake is stored separately in a vial, which takes up more space and requires dissolution before further handling, which reduces the benefits of lyophilization. A solution to this issue lies in the preparation of single-dose lyophilizates in a metal mould, on which a mixture of a filler and cryoprotectant with phage lysates (or possibly purificates) and with a solution of phage enzymes is transferred before starting the lyophilization. High stability of their antibacterial effect is thus achieved. In addition, the produced single-dose forms can be then stored in one primary package to reduce the requirements for storage space and to simplify and precise administration of the single dose at the same time.

There has been a limited number of studies dealing with single-dose solid forms containing phages and their enzymes that make it possible to precise their dosage and to increase the stability of their effect (Khanal et al., 2021). This patent is focused on the methodology of preparation of lyophilized single-dose forms containing bacteriophages and their enzymes that facilitate the administration of phages and their enzymes in the therapy of infections caused by the pathogenic strains of *P. aeruginosa* and S. *aureus.*

The above-mentioned problems with the stabilization of active ingredients in the course of preparation of lyophilized forms and the spectrum of activity are solved by the unique composition of a tablet mixture containing specific antimicrobial constituents (phages and endolysin).

### Summary of Invention

The present invention provides a mixture for the preparation of lyophilized tablets, wherein said mixture contains a phage lysate/purificate of bacteriophages acting against *Staphylococcus aureus* and *Pseudomonas aeruginosa* at the titre of 10⁷ to 10¹¹ PFU/ml, 0.01 to 0.3 mg/ml of endolysin LysMB10 produced in *E. coli* with the sequence SEQ ID NO: 1, further 5 to 50 g of a filler and 4 to 13 g of a cryoprotectant per 100 ml of the mixture.

For the preparation of tablets, the mixture is lyophilized after being applied on a mould provided with wells having the shape of the intended tablet.

The composition of the tabletting base is optimized to support the stabilization of the phages and enzymes and to prevent the reduction of their concentration and activity. This ensures the optimum efficiency of action. In addition, the combination of endolysin LysMB10 (produced by the strain *E. coli* CCM 9354) and the bacteriophages DSM 34648 (phage active against *P. aeruginosa*) and DSM 34647 (phage active against *S. aureus*) helps to considerably extend the spectrum of commonly used bacteriophages and endolysins acting against the bacteria S. *aureus* and *P. aeruginosa.* The phages DSM34648 and DSM34647 were isolated *de novo* from the environment or prepared by selective laboratory evolution and are unique in terms of their lytic spectrum. Endolysin LysMB10 is a so-far-not-described antimicrobial enzyme that acts against a wide spectrum of bacteria of the strain *Staphylococcus.*

In this invention, a specific lyophilization process of a mixture with an optimized composition that ensures stability and high activity of the antimicrobial constituents is used for the preparation of a single-dose solid form (lyophilized tablet) containing the phage lysate and endolysin. A defined mixture of active antimicrobial substances (phage lysates and endolysins), a filler (fish gelatin, maltodextrin or polyvinylpyrrolidone (PVP)) and a cryoprotectant (mannitol) is, after being applied onto a special metallic mould (Czech utility model no. CZ 31295 U1), lyophilized at defined conditions. This procedure increases the stability of the effect of the active ingredients, especially at higher temperatures, enables more convenient storage and enhances the microbiological quality of the resulting product that can be used in the treatment of bacterial infections caused by the bacteria S. *aureus* and *P. aeruginosa.* The specific phages contained in the tablet have a unique lytic spectrum, comprising pathogenic strains isolated from the hospital environment, and act synergistically with endolysin LysMB10 - which has not been described yet (has a unique amino acid sequence, included in the attached list of sequences as SEQ ID NO: 1).

### Brief Description of Drawings

### Fig.1

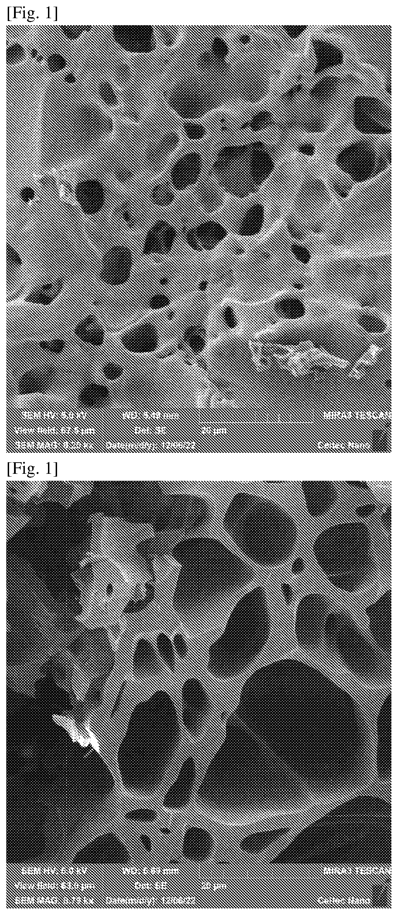

[Fig.1]: A detailed scanning electron microscopy image of the surface of a dose prepared from the specific antimicrobial mixture

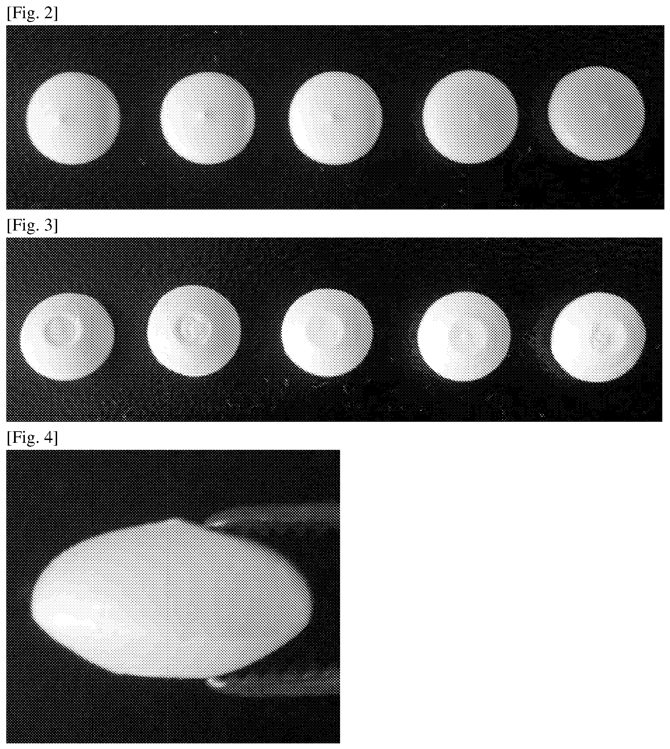

[Fig.2]: A detailed scanning electron microscopy image of the surface of a dose prepared from the specific antimicrobial mixture
[Fig.3]: A detail of the top part of a dose prepared from the specific antimicrobial mixture
[Fig.4]: A detail of the bottom part of a dose prepared from the specific antimicrobial mixture

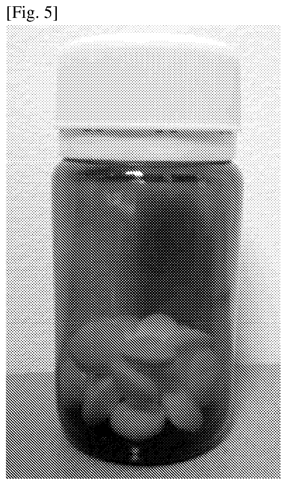

[Fig.5]: A detail of a lateral side of a dose prepared from the specific antimicrobial mixture
Figure 6: A sample of the primary package

**Sequence 1:** Primary sequence of endolysin (SEQ ID NO: 1)

### Description of Embodiments

A mixture of the phage lysate with the titre value of at least 1 x 10⁸ PFU/ml and endolysin at a concentration (0.01 - 0.3 mg/ml) can be processed into the respective quantity of solid doses (tablets). In this process, the active ingredients with the filler and cryoprotectant are frozen in a special mould in a way to produce separated solid doses which are subsequently lyophilized in the mould. The resulting lyophilized product maintains the structure of a tablet and can be stored protected from light and air humidity with the use of a suitable package at the temperature of 4°C at least for three months. For these produced doses a titre of the phage must be provided that achieves at least 10⁶ to 10⁷ PFU/ml, which is a generally recognized minimum value of phage titre for therapeutic use (Merabishvili 2014) and the activity of the endolysin, with a suitable purification method.

For the preparation, a non-purified phage lysate or a purificate with a titre value at least on the order of 1×10⁷ PFU/ml can be used. The phage lysate is purified and transferred to an SM buffer (composition of the SM buffer: 100 mM of NaCl, 8 mM of MgSO₄.7H₂O, 50 mM of Tris-Cl (pH 7.5) in 1000 ml of distilled water), which is used to store phages and at the same time it is suitable for the lyophilization process as its pH does not change during lyophilization. Commonly used methods of protein purification can be applied to the purification of phages e.g. ultracentrifugation, tangential flow filtration, chromatography or repurification with the use of centrifugation flasks with a filter adapter. The bacteriophages were isolated on strains that were not sensitive to other tested bacteriophages. These two above-mentioned bacteriophages DSM34648 and DSM34647, which are deposited in the DSMZ Collection in Germany as patent deposits, thus have a unique spectrum that extends the possibilities of using therapeutic phages to other pathogenic clinical isolates. Their nucleotide sequences are included in the attached list of sequences as SEQ ID NO: 2 (phage DSM 34647) and SEQ ID NO: 3 (phage DSM 34648). Endolysin LysMB 10, the producing *E. coli* of which is deposited in the CCM Collection in Brno as a patent deposit, represents an entirely new protein with proven antimicrobial activity against the bacteria *S. aureus* that has not been identified before. This enzyme supports the antimicrobial action of bacteriophages and against the bacteria *S. aureus*it can be even used without bacteriophages. The sequence of endolysin LysMB10 (SEQ ID NO: 1) is given separately.

For the preparation of the product, phages from the MB Pharma Collection infecting *P. aeruginosa* and *S. aureus* are used and at the same time lytic enzyme acting against S. *aureus.* The bacteriophages and the strain *E. coli* producing LysMB10 are deposited according to the Budapest Treaty in the German DSMZ Collection, or in CCM (in the case of *E. coli*) as patent deposits under the numbers CCM 9354, DSM 34648 and DSM 34647. Their basic characteristics are presented in following Tables 1 and 2:

**Table 1: Summary of phage characteristics**

| **Bacteriophage** | **Host** | **Genus** | **G+C (%)** | **Genome size (kbp)** |
|---|---|---|---|---|
| DSM 34647 | *S. aureus* | *Rosenblumvirus* | 29.24 | 18.00 |
| DSM 34648 | *P. aeruginosa* | *Septimatrevirus* | 53.74 | 42.97 |

**Table 2: Summary of characteristics of endolysin LysMB10**

| **Endolysin** | **Target organism** | **Sequence size** | **pI** | **kDa** |
|---|---|---|---|---|
| LysMB10 | *Staphylococcus* | 259 amk | 9.77 | 28.9 |

Testing of the antimicrobial effect of the phages and endolysins in a particular dose is carried out using the drop method on double-layered agar (Garbe et al. 2010) or by reduction of OD in the liquid culture (in the particular case of endolysin). In the case of the bacteriophages, before testing, one dose is dissolved in 50 ml of sterile distilled water at room temperature and 150 rpm for 3 to 5 minutes. The obtained solution is diluted and the particular dilutions are separated by dripping. A measurement is carried out for several doses to check the uniformity of their effect and at the same time stability of the antimicrobial effect of the active ingredients in time, in the ideal case at least after three months.

The impact of endolysin is monitored via the loss of turbidity (OD₆₀₀) in such a way that one tablet (originally 0.5 ml of the liquid mixture) is added to a 1 ml cuvette containing the bacterial culture. The bacterial culture was washed twice in the buffer and its density was set to the value OD₆₀₀= 0.5. Then, it was transferred to a cuvette, a tablet was put in it and the loss of OD was measured afterwards.

### Examples

The composition of the lyophilized tablets and the method of their preparation are specified in Examples 1 to 3, wherein each example differs in the composition of the mixture of the cryoprotectant and filler that are suitable for the stabilization of the bacteriophages and endolysin. The proportion of individual constituents is specified in more detail in Table 3.

**Table 3: Composition of 100 ml of a mixture for the preparation of 200 tablets containing a bacteriophage/endolysin (i.e., endolysin in mg/ml)**

| **Filler [g]** | **Cryoprotectant - mannitol [g]** | **Phage DSM 34648 [PFU /ml]** | **Phage DSM 34647 [PF U/ml]** | **Endolysin** |
|---|---|---|---|---|
| Maltodextrin 35-50 | 4-6 | 1×10⁷ - 1×10¹¹ | 1×10⁷ - 1×10¹¹ | 0.01 - 0.3 |
| Gelatin 10-15 | 11-13 | 1×10⁷ - 1x10¹¹ | 1×10⁷ - 1×10¹¹ | 0.01- 0.3 |
| PVP 5-10 | 4-6 | 1×10⁷ - 1×10¹¹ | 1×10⁷ - 1×10¹¹ | 0.01 - 0.3 |
| Maltodextrin 35-50 | 4-6 | | | 0.01 - 0.3 |
| Gelatin 10-15 | 11-13 | | | 0.01 - 0.3 |
| PVP 5-10 | 4-6 | | | 0.01 - 0.3 |

### Example 1:

43.75 g of maltodextrin and 5.00 g of mannitol are dissolved in 90 ml of a mixture of phage lysates/purificates at a concentration of at least 1 × 10⁹ PFU/ml under continuous stirring at room temperature for 30 minutes. After the dissolution of the excipients, the pH of the mixture is set to the value of 7.5 with the use of solutions of HCl and NaOH. 10 ml of a solution of the endolysin at the concentration of 0.3 mg/ml are added to the mixture. The resulting mixture is filtered through a 0.45 µm PES filter and subsequently maintained at room temperature and 150 rpm for at least 20 and at the most 30 minutes. The quantity of the mixture is prepared according to the required number of doses; one dose corresponds to 0.5 to 1 ml of the mixture, i.e. 100 to 200 doses are prepared from 100 ml of the mixture.

The mixture is applied on a sterile aluminium mould, and cooled to -80°C. During the application, the mould is maintained at a temperature of -60 to -80°C. Then, lyophilization is carried out in predetermined steps:
1. Freezing the sample at -30°C for 20 minutes
2. Primary drying at a temperature of -30°C for 960 minutes (i.e. 16 hours) and a pressure of 19.99 Pa (150 mTorr)
3. Secondary drying is carried out from - 30°C to 20°C at the temperature gradient of 0.1°C/min and a pressure of 19.99 Pa (150 mTorr)

After being removed from the lyophilizer and the mould, the obtained doses (tablets) are transferred into the primary package and are stored optimally at the temperature of 4°C (or at room temperature).

### Example 2:

Fish gelatin can also be used as the filler, wherein 12 g of mannitol and 12 g of fish gelatin are heated up to approximately 70°C in 50 ml of water under continuous stirring. This mixture is subsequently set to 50°C and after the setting, the phage lysates/purificates (Table 3) are admixed to it up to the total volume of 90 ml. The concentration of the phages is at least 1 × 10⁹ PFU/ml. The mixture is stirred at room temperature for 30 minutes. After the dissolution of the excipients, the pH of the mixture is set to the value of 7.5 with the use of solutions of HCl and NaOH. After adjustment of the pH of the mixture, 10 ml of a solution of the endolysin are added to make the final concentration of endolysin 0.02 mg/ml. The final mixture is filtered through a 0.45 µm PES filter and subsequently, it is maintained at room temperature and 150 rpm for at least 20 and at the most 30 minutes. The quantity of the mixture is prepared according to the required number of doses, one dose corresponds to 0.5 to 1 ml of the mixture, i.e. 100 to 200 doses are prepared from 100 ml of the mixture.

The mixture is applied on a sterile aluminium mould, and cooled to -80°C. During the application, the mould is maintained at a temperature of -60 to -80°C. Then, lyophilization is carried out in predetermined steps:
1. Freezing the sample at -30°C for 20 minutes
2. Primary drying at a temperature of -30°C for 960 minutes (i.e. 16 hours) and a pressure of 19.99 Pa (150 mTorr)
3. Secondary drying is carried out from - 30°C to 20°C at the temperature gradient of 0.1°C/min and a pressure of 19.99 Pa (150 mTorr)

After being removed from the lyophilizer and the mould, the obtained doses (tablets) are transferred into the primary package and are stored optimally at the temperature of 4°C (or at room temperature).

### Example 3:

Polyvinylpyrrolidone K90 (PVP90) can be used as the filler. 7.5 g of PVP90 and 5.00 g of mannitol are dissolved in 90 ml of a mixture of phage lysates/purificates at a concentration of at least 1 × 10⁹ PFU/ml under continuous stirring at room temperature for 30 minutes. After the dissolution of the excipients, the pH of the mixture is set to the value of 7.5 with the use of solutions of HCl and NaOH. After adjustment of the pH of the mixture, 10 ml of a solution of the endolysin are added to make the final concentration of endolysin 0.02 mg/ml. The final mixture is filtered through a 0.45 µm PES filter and subsequently, it is maintained at room temperature and 150 rpm for at least 20 and at the most 30 minutes. The quantity of the mixture is prepared according to the required number of doses, one dose corresponds to 0.5 to 1 ml of the mixture, i.e. 100 to 200 doses are prepared from 100 ml of the mixture.

The mixture is applied on a mould, and cooled to -80°C. During the application, the mould is maintained at a temperature of -60 to -80°C. Then, lyophilization is carried out in predetermined steps:
1. Freezing the sample at -30°C for 20 minutes
2. Primary drying at a temperature of -30°C for 960 minutes (i.e. 16 hours) and a pressure of 19.99 Pa (150 mTorr)
3. Secondary drying is carried out from - 30°C to 20°C at the temperature gradient of 0.1°C/min and a pressure of 19.99 Pa (150 mTorr)

After being removed from the lyophilizer and the mould, the obtained doses (tablets) are transferred into the primary package and are stored optimally at temperature of 4°C (or at the room temperature).

### Example 4:

43.75 g of maltodextrin and 5.00 g of mannitol are dissolved in 90 ml of a mixture of phage lysates/purificates at a concentration of at least 1 × 10⁹ PFU/ml under continuous stirring at room temperature for 30 minutes. After the dissolution of the excipients, the pH of the mixture is set to the value of 7.5 with the use of solutions of HCl and NaOH. After adjustment of the pH of the mixture, 10 ml of a solution of the endolysin are added to make the final concentration of endolysin 0.02 mg/ml. The final mixture is filtered through a 0.45 µm PES filter and subsequently, it is maintained at room temperature and 150 rpm for at least 20 and at the most 30 minutes. The quantity of the mixture is prepared according to the required number of doses, one dose corresponds to 0.5 to 1 ml of the mixture, i.e. 100 to 200 doses are prepared from 100 ml of the mixture.

The mixture is applied on a mould, and cooled to -80°C. During the application, the mould is maintained at a temperature of -60 to -80°C. Then, lyophilization is carried out in predetermined steps:
1. Freezing the sample at -30°C for 20 minutes
2. Primary drying at a temperature of -30°C for 960 minutes (i.e. 16 hours) and a pressure of 19.99 Pa (150 mTorr)
3. Secondary drying is carried out from - 30°C to 20°C at the temperature gradient of 0.1°C/min and a pressure of 19.99 Pa (150 mTorr)

After being removed from the lyophilizer and the mould, the obtained doses (tablets) are transferred into the primary package and are stored optimally at the temperature of 4°C (or at room temperature).

### Industrial Applicability

Lyophilized single-dose forms (tablets) containing a phage lysate/purificate and endolysin prepared from the concerned mixture can be applied within the phage therapy of infections caused by a wide spectrum of the strains of pathogenic bacteria S. *aureus* and *P. aeruginosa,* which are provably sensitive to these active ingredients.

### Reference to Deposited Biological Material

The bacteriophages and the strain *E. coli* producing LysMB10 are deposited according to the Budapest Treaty in the German DSMZ Collection, or in CCM (in the case of *E. coli*) as patent deposits under the numbers CCM 9354, DSM 34648 and DSM 34647.

### Citation List

Abedon, Stephen T., Sarah J. Kuhl, Bob G. Blasdel, and Elizabeth Martin Kutter. 2011. Phage Treatment of Human Infections. Bacteriophage 1 (2): 66-85. https:/ doi.org/10.4161/bact.1.2.15845.
Abdelrahman, F.; Easwaran, M.; Daramola, O.I.; Ragab, S.; Lynch, S.; Oduselu, T.J.; Khan, F.M.; Ayobami, A.; Adnan, F.; Torrents, E.; et al. 2021. Phage-Encoded Endolysins. Antibiotics 10 (124): https:/doi.org/10.3390/ antibiotics10020124
Anany, H., Chen, W., Pelton, R., & Griffiths, M. W. (2011). Biocontrol of Listeria monocytogenes and Escherichia coli O157:H7 in meat by using phages immobilized on modified cellulose membranes. Applied and Environmental Microbiology, 77(18), 6379-6387. https:/doi.org/10.1128/AEM.05493-11
Barer a Irving. Medical Microbiology, 19th Edition: A Guide to Microbial Infections: Pathogenesis, Immunity, Laboratory Investigation and Control. 19. United Kingdom: Elsevier, 2018. ISBN 978-0-7020-7200-0.
Brown, T. L., Petrovski, S., Chan, H. T., Angove, M. J., & Tucci, J. (2018). Semi-solid and solid dosage forms for the delivery of phage therapy to epithelia. Pharmaceuticals, 11(1), 1-12. https:/doi.org/10.3390/ph11010026
Carlson, K. 2005. Working with bacteriophages: Common techniques and methodological approaches In: Kutter, E., Sulakvelidze, A, (ed.) Bacteriophages: Biology and Application, p. 437-494. CRC Press, Boca Raton, FL, USA
Clark, William A. 1962. "Comparison of Several Methods for Preserving Bacteriophages," no. 1951.
Fulgione, A., Ianniello, F., Papaianni, M., Contaldi, F., Sgamma, T., Giannini, C., Pastore, S., Velotta, R., Ventura, B. Della, Roveri, N., Lelli, M., Capuano, F., & Capparelli, R. (2019). Biomimetic hydroxyapatite nanocrystals are an active carrier for salmonella bacteriophages. International Journal of Nanomedicine, 14, 2219-2232. https:/doi.org/10.2147/IJN.S190188
Garbe, Julia, Andrea Wesche, Boyke Bunk, Marlon Kazmierczak, Katherina Selezska, Christine Rohde, Johannes Sikorski, Manfred Rohde, Dieter Jahn, and Max Schobert. 2010. Characterization of JG024, a Pseudomonas Aeruginosa PB1-like Broad Host Range Phage under Simulated Infection Conditions. BMC Microbiology 10: 1-10. https:/doi.org/10.1186/1471-2180-10-301
Hoe, Susan, James W. Ivey, Mohammed A. Boraey, Abouzar Shamsaddini-Shahrbabak, Emadeddin Javaheri, Sadaf Matinkhoo, Warren H. Finlay, and Reinhard Vehring. 2014. Use of a Fundamental Approach to Spray-Drying Formulation Design to Facilitate the Development of Multi-Component Dry Powder Aerosols for Respiratory Drug Delivery. Pharmaceutical Research 31 (2): 449-65. https:/doi.org/10 .1007/s11095-013-1174-5.
Jault, P., Leclerc, T., Jennes, S., Pirnay, J. P., Que, Y. A., Resch, G., Rousseau, A. F., Ravat, F., Carsin, H., Le Floch, R., Schaal, J. V., Soler, C., Fevre, C., Arnaud, I., Bretaudeau, L., & Gabard, J. (2019). Efficacy and tolerability of a cocktail of bacteriophages to treat burn wounds infected by Pseudomonas aeruginosa (PhagoBurn): a randomised, controlled, double-blind phase 1/2 trial. The Lancet Infectious Diseases, 19(1), 35-45. https:/doi.org/10.1016/S1473-3099(18)30482-1
Khanal, D., Chang, R. Y. K., Hick, C., Morales, S., & Chan, H.-K. (2021). Enteric-coated bacteriophage tablets for oral administration against gastrointestinal infections. International Journal of Pharmaceutics, 609(July), 121206. https:/doi.org/10.1016/ j.ijpharm.2021.121206
Komárek, Pavel a Miloslava Rabišková. Technologie lék 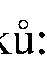 : galenika. 3., přeprac. a dopl. vyd. Praha: Galén, c2006. ISBN 80-726-2423-7.
Kropinski, A.M., Mazzocco, A., Waddell, T.E., Lingohr, E., Johnson, R.P. 2009 Enumeration of bacteriophages by double agar overlay plaque assay. In: Clokie M.R., Kropinski A.M. (ed.) Bacteriophages: Methods and Protocols, Vol 1: Isolation, Characterization, and Interactions. pp. 69-67. doi.org/10.1007/978-1-60327-164-6_7.
Loc-Carrillo, Catherine, and Stephen T. Abedon. 2011. Pros and Cons of Phage Therapy. Bacteriophage 1 (2): 111-14. https:/doi.org/10.4161/bact.1.2.14590.
Malik, Danish J. 2021. Approaches for Manufacture, Formulation, Targeted Delivery and Controlled Release of Phage-Based Therapeutics. Current Opinion in Biotechnology 68: 262-71. https:/doi.org/10.1016/j.copbio.2021.02.009
Malik, Danish J., Ilya J. Sokolov, Gurinder K. Vinner, Francesco Mancuso, Salvatore Cinquerrui, Goran T. Vladisavljevic, Martha R.J. Clokie, Natalie J. Garton, Andrew G.F. Stapley, and Anna Kirpichnikova. 2017. Formulation, Stabilisation and Encapsulation of Bacteriophage for Phage Therapy. Advances in Colloid and Interface Science 249 (May): 100-133. https:/doi.org/10.1016/j.cis.2017.05.014.
Mathias, J. R., Dodd, M. E., Walters, K. B., Yoo, S. K., Erik, A., & Huttenlocher, A. (2010). Phage-Bacterium War on Polymeric Surfaces-Anchored Eliminate Micobial Infections. Biomacromolecules, 33(11), 1212-1217. https:/doi.org/10.1021/ bm400290u.Phage-Bacterium
Merabishvili M. Production of bacteriophages using bacterial suspension cultures for phage-therapy. In: Meyer HP, Schmidhalter DR, editors. Industrial scale suspension culture of living cells. 2014. Wiley VCH Verlag, Weinheim, Germany; 2014. pp. 537-543.
Meyer HP, Schmidhalter DR, editors. Industrial scale suspension culture of living cells. 2014. WileyVCH Verlag, Weinheim, Germany; 2014. pp. 537-543.
Subils, T., Aquili, V., Ebner, G., & Balagué, C. (2012). Effect of preservatives on Shiga toxigenic phages and Shiga toxin of Escherichia coli O157:H7. Journal of Food Protection, 75(5), 959-965. https:/doi.org/10.4315/0362-028X.JFP-11-332
URL 1: Antimicrobial resistance, 17. 11. 2021,
https:/www.who.int/news-room/fact-sheets/detail/antimicrobial-resistance

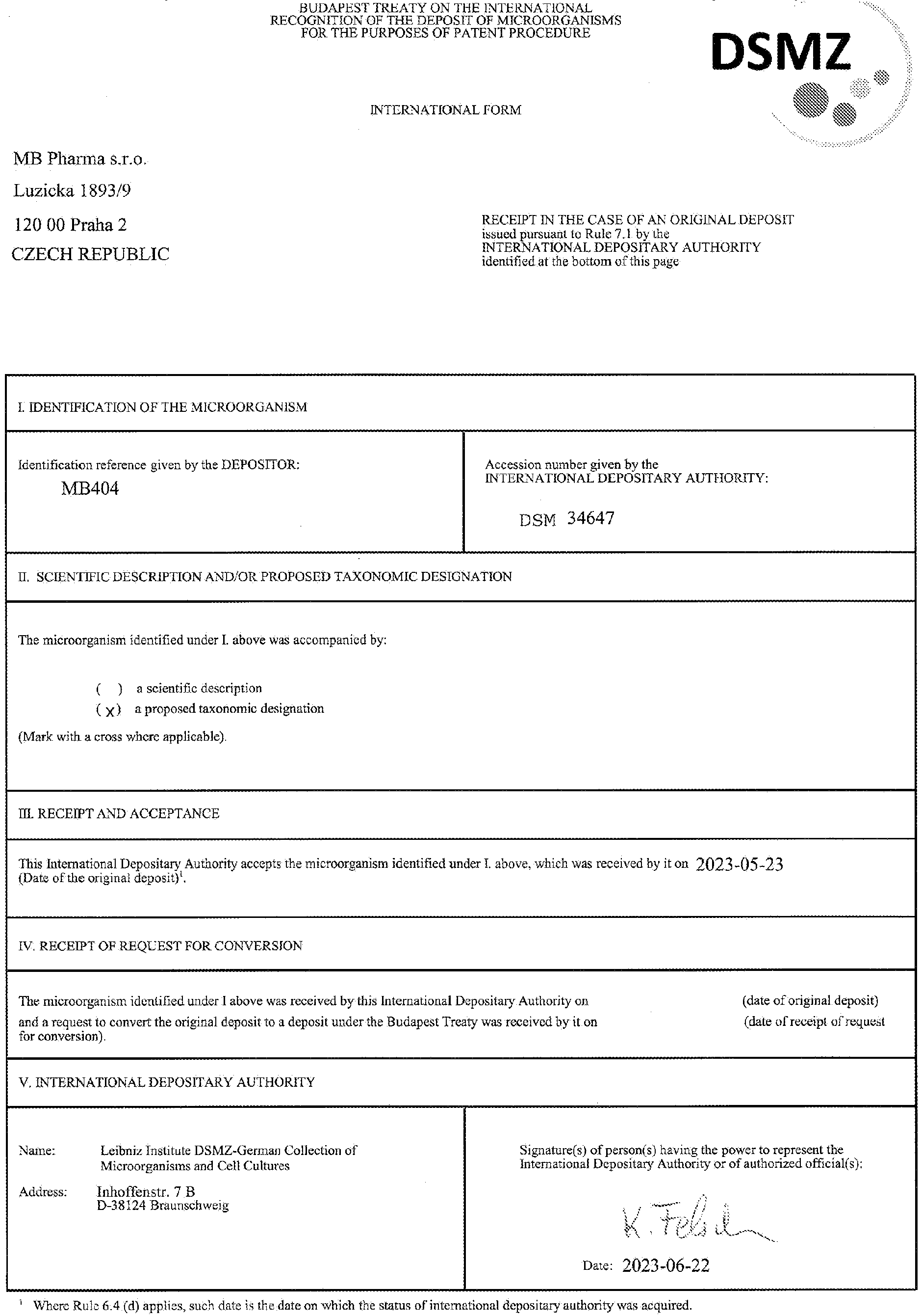
BUDAPEST TREATY ON THE INTERNATIONAL
RECOGNITION OF THE DEPOSIT OF MICROORGANISMS
FOR THE PURPOSES OF PATENT PROCEDURE

DSMZ

INTERNATIONAL FORM

MB Pharma s.r.o.
Luzicka 1893/9
120 00 Praha 2
CZECH REPUBLIC

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR:
MB404 | Accession number given by the
INTERNATIONAL DEPOSITARY AUTHORITY:
DSM 34647

II. SCIENTIFIC DESCRIPTION AND/OR PROPOSED TAXONOMIC DESIGNATION

The microorganism identified under I. above was accompanied by:

( ) a scientific description
( x ) a proposed taxonomic designation

(Mark with a cross where applicable).

III. RECEIPT AND ACCEPTANCE

This International Depositary Authority accepts the microorganism identified under I. above, which was received by it on 2023-05-23
(Date of the original deposit)¹.

IV. RECEIPT OF REQUEST FOR CONVERSION

The microorganism identified under I above was received by this International Depositary Authority on (date of original deposit)
and a request to convert the original deposit to a deposit under the Budapest Treaty was received by it on (date of receipt of request
for conversion).

V. INTERNATIONAL DEPOSITARY AUTHORITY

Name: Leibniz Institute DSMZ-German Collection of
Microorganisms and Cell Cultures
Address: Inhoffenstr. 7 B
D-38124 Braunschweig | Signature(s) of person(s) having the power to represent the
International Depositary Authority or of authorized official(s):
Date: 2023-06-22

¹ Where Rule 6.4 (d) applies, such date is the date on which the status of international depositary authority was acquired.

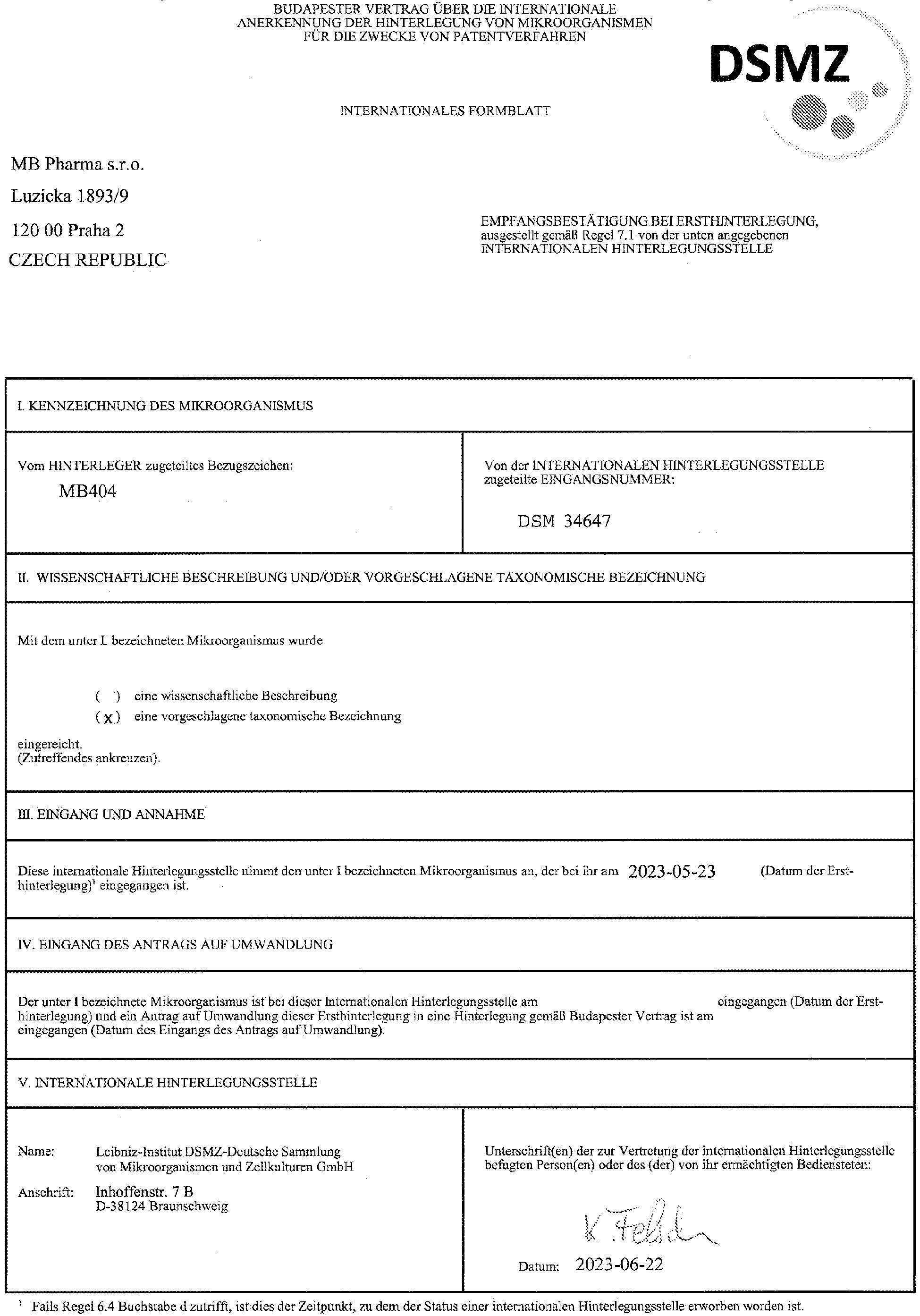
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

DSMZ

INTERNATIONALES FORMBLATT

MB Pharma s.r.o.
Luzicka 1893/9
120 00 Praha 2
CZECH REPUBLIC

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen:
MB404 | Von der INTERNATIONALEN HINTERLEGUNGSSTELLE
zugeteilte EINGANGSNUMMER:
DSM 34647

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

( ) eine wissenschaftliche Beschreibung
( x ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 2023-05-23 (Datum der Erst-
hinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: Leibniz-Institut DSMZ-Deutsche Sammlung
von Mikroorganismen und Zellkulturen GmbH

Anschrift: Inhoffenstr. 7 B
D-38124 Braunschweig | Unterschrift(en) der zur Vertretung der internationalen Hinterlegungsstelle
befugten Person(en) oder des (der) von ihr ermächtigten Bediensteten:


Datum: 2023-06-22

¹ Falls Regel 6.4 Buchstabe d zutrifft, ist dies der Zeitpunkt, zu dem der Status einer internationalen Hinterlegungsstelle erworben worden ist.

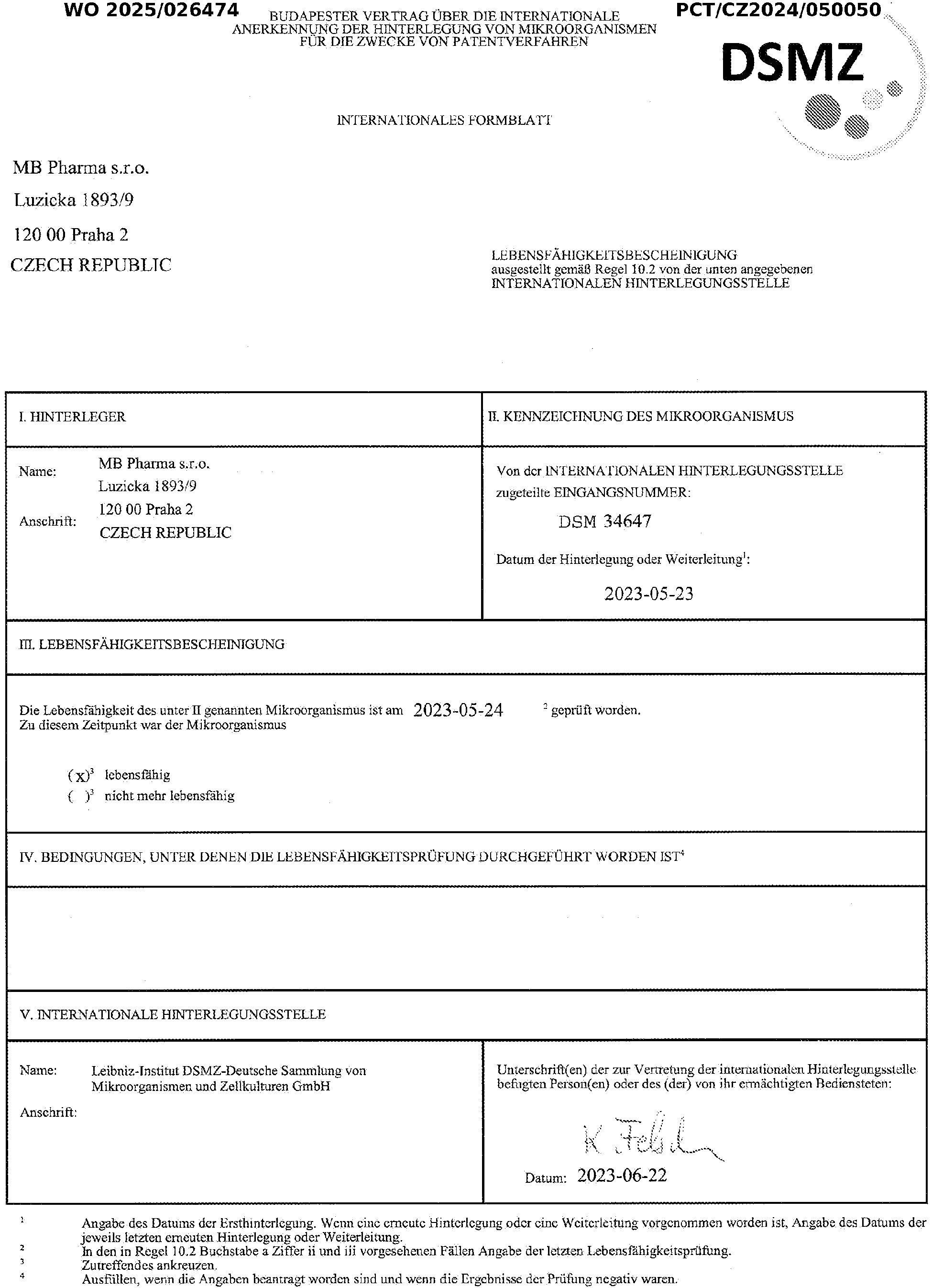
WO 2025/026474
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN
PCT/CZ2024/050050
DSMZ

INTERNATIONALES FORMBLATT

MB Pharma s.r.o.
Luzicka 1893/9
120 00 Praha 2
CZECH REPUBLIC

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: MB Pharma s.r.o.
Luzicka 1893/9
Anschrift: 120 00 Praha 2
CZECH REPUBLIC | Von der INTERNATIONALEN HINTERLEGUNGSSTELLE
zugeteilte EINGANGSNUMMER:
DSM 34647
Datum der Hinterlegung oder Weiterleitung¹:
2023-05-23

III. LEBENSFÄHIGKEITSBESCHEINIGUNG

Die Lebensfähigkeit des unter II genannten Mikroorganismus ist am 2023-05-24 ² geprüft worden.
Zu diesem Zeitpunkt war der Mikroorganismus

(x)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: Leibniz-Institut DSMZ-Deutsche Sammlung von
Mikroorganismen und Zellkulturen GmbH
Anschrift: | Unterschrift(en) der zur Vertretung der internationalen Hinterlegungsstelle
befugten Person(en) oder des (der) von ihr ermächtigten Bediensteten:
Datum: 2023-06-22

¹ Angabe des Datums der Ersthinterlegung. Wenn eine erneute Hinterlegung oder eine Weiterleitung vorgenommen worden ist, Angabe des Datums der
jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

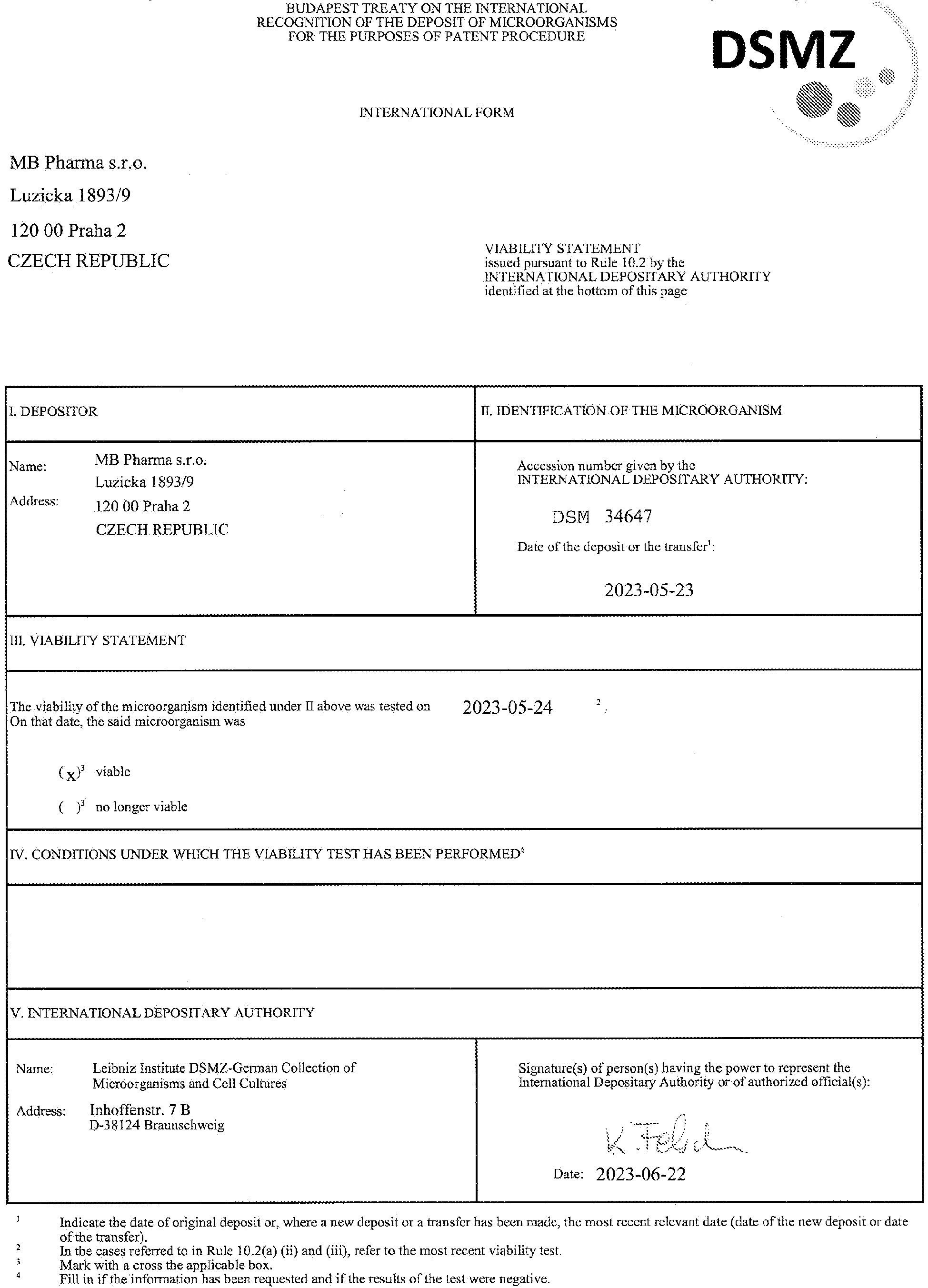
BUDAPEST TREATY ON THE INTERNATIONAL
RECOGNITION OF THE DEPOSIT OF MICROORGANISMS
FOR THE PURPOSES OF PATENT PROCEDURE

DSMZ

INTERNATIONAL FORM

MB Pharma s.r.o.
Luzicka 1893/9
120 00 Praha 2
CZECH REPUBLIC

VIABILITY STATEMENT
issued pursuant to Rule 10.2 by the
INTERNATIONAL DEPOSITARY AUTHORITY
identified at the bottom of this page

I. DEPOSITOR | II. IDENTIFICATION OF THE MICROORGANISM
Name: MB Pharma s.r.o.
Luzicka 1893/9
Address: 120 00 Praha 2
CZECH REPUBLIC | Accession number given by the
INTERNATIONAL DEPOSITARY AUTHORITY:
DSM 34647
Date of the deposit or the transfer¹:
2023-05-23

III. VIABILITY STATEMENT

The viability of the microorganism identified under II above was tested on 2023-05-24 ².
On that date, the said microorganism was

(x)³ viable
( )³ no longer viable

IV. CONDITIONS UNDER WHICH THE VIABILITY TEST HAS BEEN PERFORMED⁴

V. INTERNATIONAL DEPOSITARY AUTHORITY

Name: Leibniz Institute DSMZ-German Collection of
Microorganisms and Cell Cultures
Address: Inhoffenstr. 7 B
D-38124 Braunschweig | Signature(s) of person(s) having the power to represent the
International Depositary Authority or of authorized official(s):
Date: 2023-06-22

¹ Indicate the date of original deposit or, where a new deposit or a transfer has been made, the most recent relevant date (date of the new deposit or date of the transfer).
² In the cases referred to in Rule 10.2(a) (ii) and (iii), refer to the most recent viability test.
³ Mark with a cross the applicable box.
⁴ Fill in if the information has been requested and if the results of the test were negative.

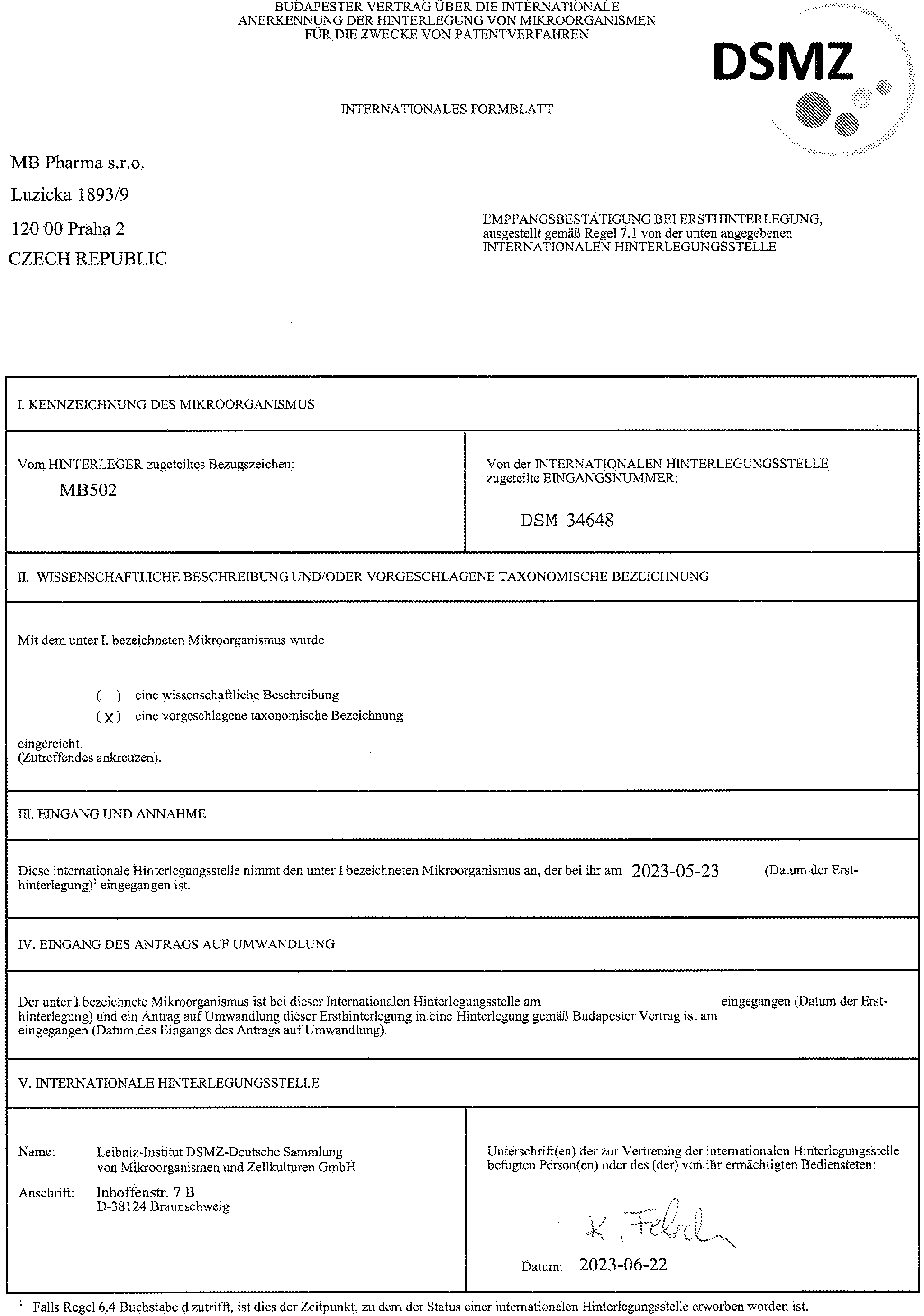
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

DSMZ

INTERNATIONALES FORMBLATT

MB Pharma s.r.o.
Luzicka 1893/9
120 00 Praha 2
CZECH REPUBLIC

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen:
MB502 | Von der INTERNATIONALEN HINTERLEGUNGSSTELLE
zugeteilte EINGANGSNUMMER:
DSM 34648

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

( ) eine wissenschaftliche Beschreibung
( X ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 2023-05-23 (Datum der Erst-
hinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: Leibniz-Institut DSMZ-Deutsche Sammlung
von Mikroorganismen und Zellkulturen GmbH
Anschrift: Inhoffenstr. 7 B
D-38124 Braunschweig | Unterschrift(en) der zur Vertretung der internationalen Hinterlegungsstelle
befugten Person(en) oder des (der) von ihr ermächtigten Bediensteten:
Datum: 2023-06-22

¹ Falls Regel 6.4 Buchstabe d zutrifft, ist dies der Zeitpunkt, zu dem der Status einer internationalen Hinterlegungsstelle erworben worden ist.

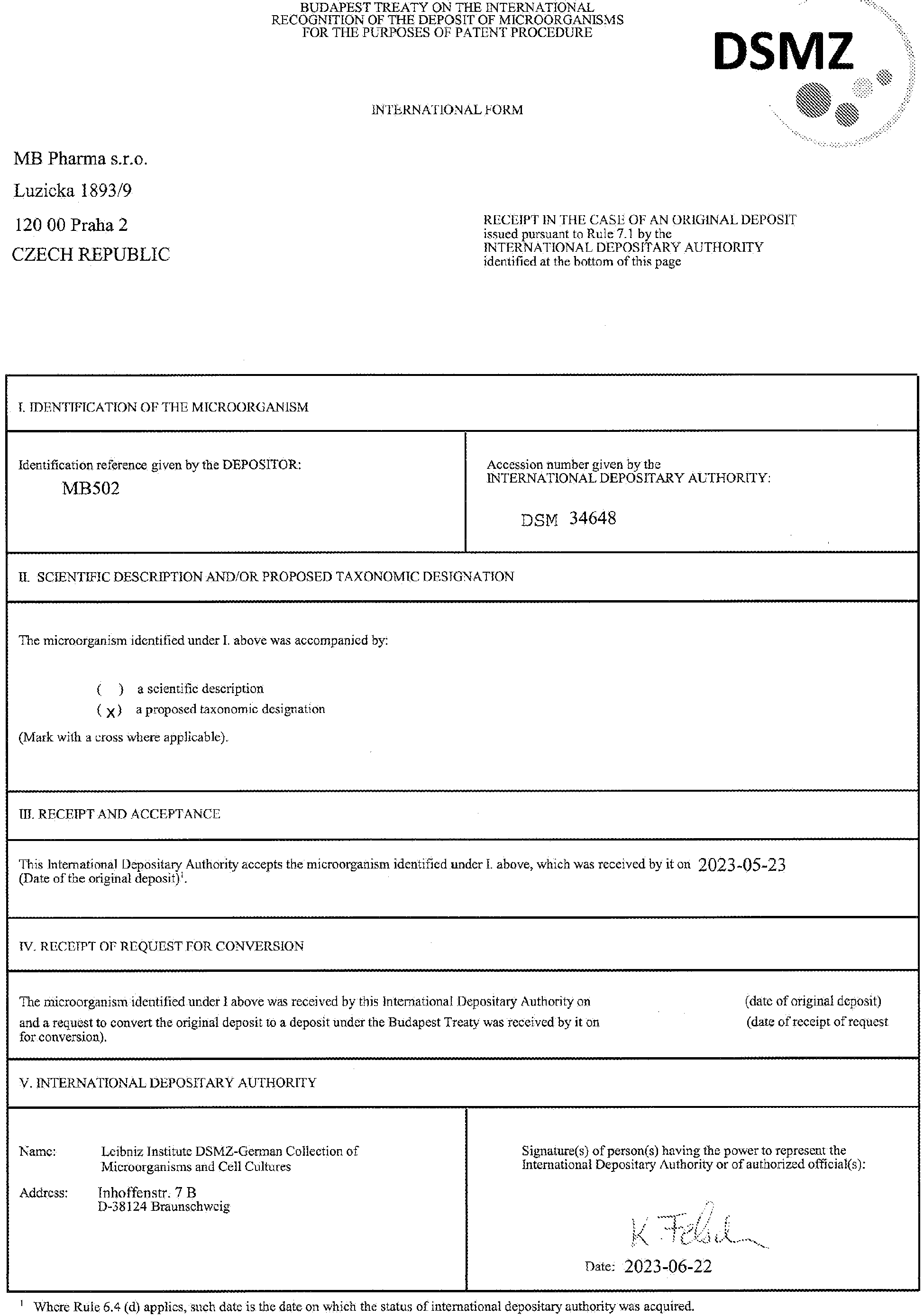
BUDAPEST TREATY ON THE INTERNATIONAL
RECOGNITION OF THE DEPOSIT OF MICROORGANISMS
FOR THE PURPOSES OF PATENT PROCEDURE

DSMZ

INTERNATIONAL FORM

MB Pharma s.r.o.
Luzicka 1893/9
120 00 Praha 2
CZECH REPUBLIC

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR: | Accession number given by the
MB502 | INTERNATIONAL DEPOSITARY AUTHORITY:
 | DSM 34648

II. SCIENTIFIC DESCRIPTION AND/OR PROPOSED TAXONOMIC DESIGNATION

The microorganism identified under I. above was accompanied by:

( ) a scientific description
( x ) a proposed taxonomic designation

(Mark with a cross where applicable).

III. RECEIPT AND ACCEPTANCE

This International Depositary Authority accepts the microorganism identified under I. above, which was received by it on 2023-05-23
(Date of the original deposit)¹.

IV. RECEIPT OF REQUEST FOR CONVERSION

The microorganism identified under I above was received by this International Depositary Authority on (date of original deposit)
and a request to convert the original deposit to a deposit under the Budapest Treaty was received by it on (date of receipt of request
for conversion).

V. INTERNATIONAL DEPOSITARY AUTHORITY

Name: Leibniz Institute DSMZ-German Collection of
Microorganisms and Cell Cultures
Address: Inhoffenstr. 7 B
D-38124 Braunschweig | Signature(s) of person(s) having the power to represent the
International Depositary Authority or of authorized official(s):
Date: 2023-06-22

¹ Where Rule 6.4 (d) applies, such date is the date on which the status of international depositary authority was acquired.

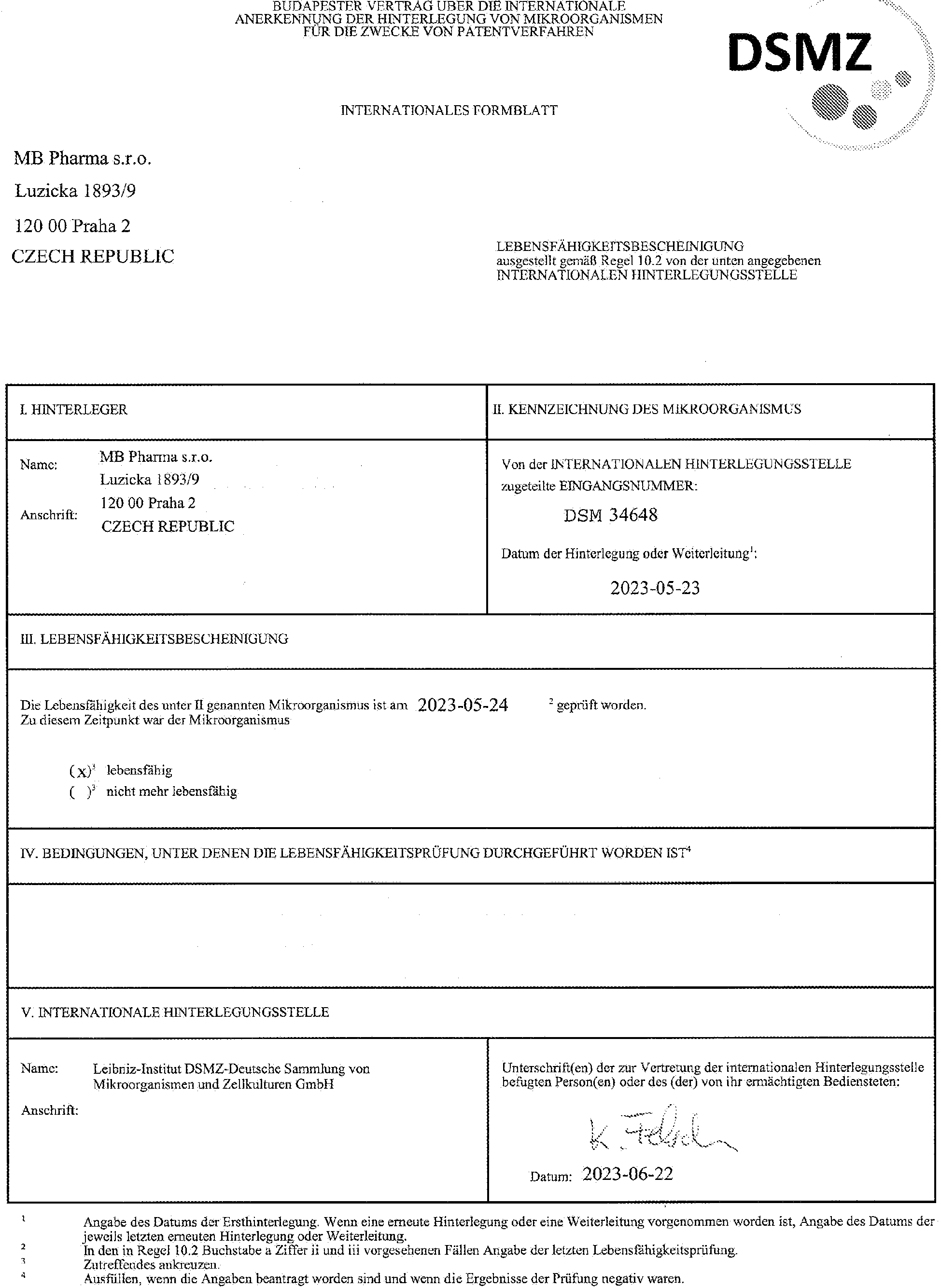
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

DSMZ

INTERNATIONALES FORMBLATT

MB Pharma s.r.o.
Luzicka 1893/9
120 00 Praha 2
CZECH REPUBLIC

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: MB Pharma s.r.o.
Luzicka 1893/9
Anschrift: 120 00 Praha 2
CZECH REPUBLIC | Von der INTERNATIONALEN HINTERLEGUNGSSTELLE
zugeteilte EINGANGSNUMMER:
DSM 34648
Datum der Hinterlegung oder Weiterleitung¹:
2023-05-23

III. LEBENSFÄHIGKEITSBESCHEINIGUNG

Die Lebensfähigkeit des unter II genannten Mikroorganismus ist am 2023-05-24 ² geprüft worden.
Zu diesem Zeitpunkt war der Mikroorganismus

(x)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: Leibniz-Institut DSMZ-Deutsche Sammlung von
Mikroorganismen und Zellkulturen GmbH
Anschrift: | Unterschrift(en) der zur Vertretung der internationalen Hinterlegungsstelle
befugten Person(en) oder des (der) von ihr ermächtigten Bediensteten:
Datum: 2023-06-22

¹ Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

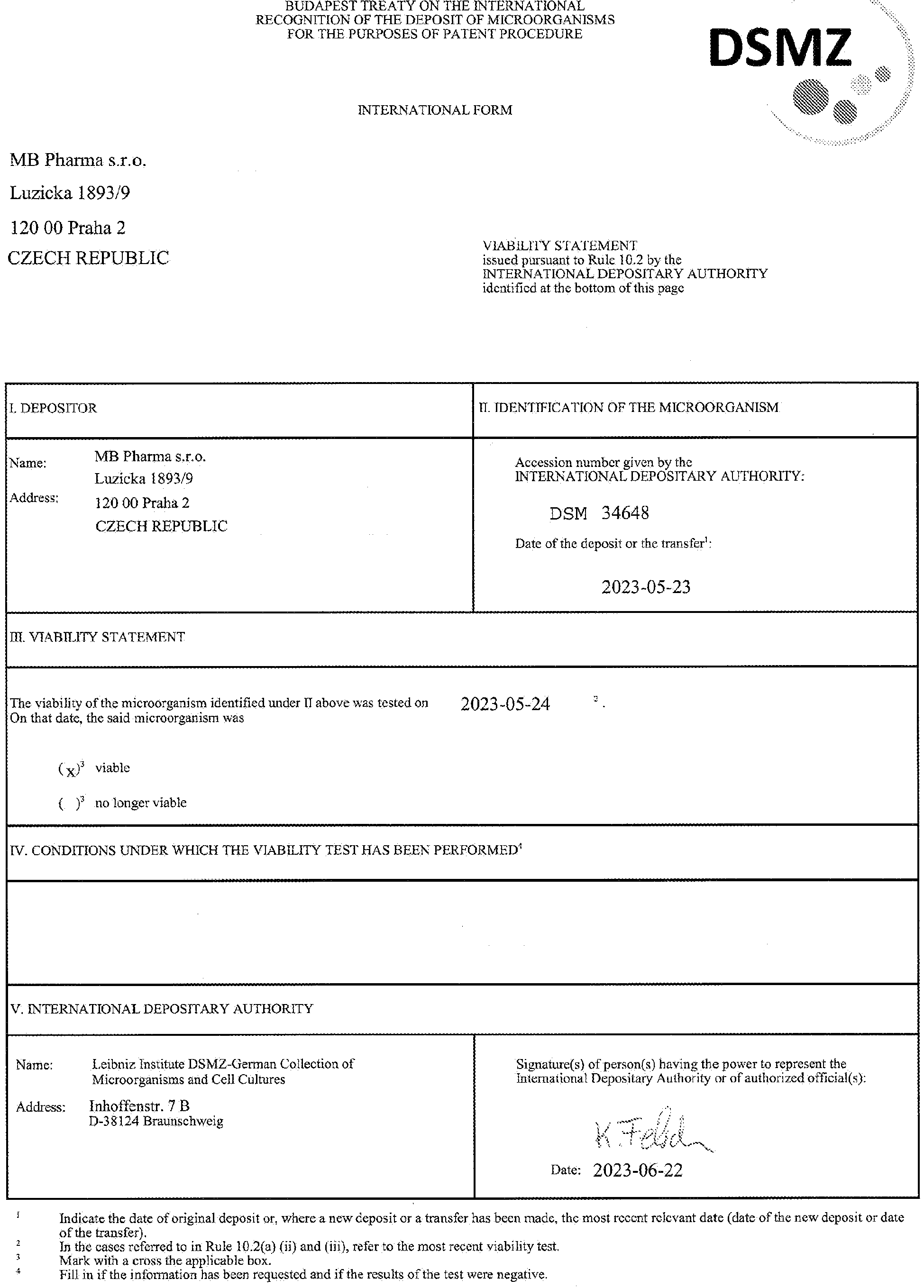
BUDAPEST TREATY ON THE INTERNATIONAL
RECOGNITION OF THE DEPOSIT OF MICROORGANISMS
FOR THE PURPOSES OF PATENT PROCEDURE

DSMZ

INTERNATIONAL FORM

MB Pharma s.r.o.
Luzicka 1893/9
120 00 Praha 2
CZECH REPUBLIC

VIABILITY STATEMENT
issued pursuant to Rule 10.2 by the
INTERNATIONAL DEPOSITARY AUTHORITY
identified at the bottom of this page

I. DEPOSITOR | II. IDENTIFICATION OF THE MICROORGANISM
Name: MB Pharma s.r.o.
Luzicka 1893/9
Address: 120 00 Praha 2
CZECH REPUBLIC | Accession number given by the
INTERNATIONAL DEPOSITARY AUTHORITY:
DSM 34648
Date of the deposit or the transfer1:
2023-05-23

III. VIABILITY STATEMENT

The viability of the microorganism identified under II above was tested on 2023-05-24 2.
On that date, the said microorganism was

(x)3 viable
( )3 no longer viable

IV. CONDITIONS UNDER WHICH THE VIABILITY TEST HAS BEEN PERFORMED4

V. INTERNATIONAL DEPOSITARY AUTHORITY

Name: Leibniz Institute DSMZ-German Collection of
Microorganisms and Cell Cultures
Address: Inhoffenstr. 7 B
D-38124 Braunschweig | Signature(s) of person(s) having the power to represent the
International Depositary Authority or of authorized official(s):
Date: 2023-06-22

1 Indicate the date of original deposit or, where a new deposit or a transfer has been made, the most recent relevant date (date of the new deposit or date of the transfer).
2 In the cases referred to in Rule 10.2(a) (ii) and (iii), refer to the most recent viability test.
3 Mark with a cross the applicable box.
4 Fill in if the information has been requested and if the results of the test were negative.

WO 2025/026474 PCT/CZ2024/050050
MUNI of Experimental Biology
SCI Czech Collection
of Microorganisms

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

DEPOSITOR:

RNDr. Marek Moša, Ph.D.
MB PHARMA s.r.o.
Lužická 1893/9
120 00 Praha 2

RECEIPT IN THE CASE OF AN ORIGINAL DEPOSIT
issued pursuant to Rule 7.1 by the
INTERNATIONAL DEPOSITARY AUTHORITY (IDA)
identified at the bottom of this page

I. IDENTIFICATION OF THE MICROORGANISM
Identification reference given by the DEPOSITOR: | Accession number given by the IDA:
Escherichia coli BL21(DE3) LysMB10 | CCM 9354

II. SCIENTIFIC DESCRIPTION AND/OR TAXONOMIC DESIGNATION
The microorganism identified under part I. above was accompanied by:
☐ a scientific description
☒ a proposed taxonomic designation

III. RECEIPT AND ACCEPTANCE

The Czech Collection of Microorganisms in its capacity of an international depositary authority accepts the
microorganism listed under part I. above, which was received by IDA on 11 July, 2023 (date of the original deposit)¹

IV. RECEIPT OF THE REQUEST FOR CONVERSION

The microorganism identified under part I. above, which was received by this IDA on (date of the original deposit)
and the request to convert the original deposit to the deposit under the Budapest Treaty was received by IDA on
(date of receipt of a request for conversion)

V. INTERNATIONAL DEPOSITARY AUTHORITY (IDA)
Name: Czech Collection of Microorganisms | Signature of the person authorised to represent the
Department of Experimental Biology, | International Depositary Authority:
Faculty of Science, Masaryk University |

Address: Kamenice 5, 625 00 Brno, Czech Republic | doc. RNDr. Pavel Švec, Ph.D.

tel. +420 549 491 430 |
e-mail: ccm@sci.muni.cz | Date: 9.8.2023 Czech Collection of Microorganisms Masaryk University 625 00 BRNO, Kamenice 5 CZECH REPUBLIC

¹Where Rule 6.4 (d) applies, such date is the date on which the status of IDA was acquired.

Masaryk University, Faculty of Science
Department of Experimental Biology, Czech Collection of Microorganisms

IF 91.05 Kamenice 5, building E25, 625 00 Brno, Czech Republic
1/1 T: +420 549 49 1430, E: ccm@sci.muni.cz, https://ccm.sci.muni.cz

WO 2025/026474 PCT/CZ2024/050050
M U N I of Experimental Biology
S C I Czech Collection
of Microorganisms

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

VIABILITY STATEMENT
issued pursuant to Rule 10.2 by the INTERNATIONAL DEPOSITARY AUTHORITY (IDA)
identified at the bottom of this page

I. DEPOSITOR | II. IDENTIFICATION OF THE MICROORGANISM
Name: RNDr. Marek Moša, Ph.D.

Address:
MB PHARMA s.r.o.
Lužická 1893/9
120 00 Praha 2 | Name: Escherichia coli BL21(DE3) LysMB10

Accession number given by the IDA:

CCM 9354

Date of the deposit or transfer¹: 11 July, 2023
III. VIABILITY STATEMENT
The viability of the microorganism specified above was tested on²: 27 July, 2023

At that time the tested microorganism was:

☒ viable

☐ no longer viable
IV. CONDITIONS OF THE VIABILITY TEST³

V. INTERNATIONAL DEPOSITARY AUTHORITY
Name: Czech Collection of Microorganisms
Department of Experimental Biology,
Faculty of Science, Masaryk University

Address: Kamenice 5, 625 00 Brno, Czech Republic

tel. +420 549 491 430
e-mail: ccm@sci.muni.cz | Signature of the person authorised to represent
the International Depositary Authority:

doc. RNDr. Pavel Švec, Ph.D.

Date: 9.8.2023
Czech Collection of Microorganisms
Masaryk University
625 00 BRNO, Kamenice 5
CZECH REPUBLIC

¹ Indicate the date of the original deposit or in case of a new deposit or a new transfer indicate the most recent relevant date.
² In the cases referred to the Rule 10.2(a) (ii) and (iii) indicate the most recent viability test.
³ To be filled in only if the information on the testing conditions was requested and/or the viability test was negative.

Masaryk University, Faculty of Science
Department of Experimental Biology, Czech Collection of Microorganisms

IF 89.05
1/1
Kamenice 5, building E25, 625 00 Brno, Czech Republic
T: +420 549 49 1430, E: ccm@sci.muni.cz, https://ccm.sci.muni.cz

## Claims

1. A mixture for the preparation of lyophilized tablets with an antimicrobial effect against the bacteria *Staphylococcus aureus* and *Pseudomonas aeruginosa* containing bacteriophages and lytic enzymes with an optimum stability and efficiency of the active ingredients, **characterized in that** it contains a phage lysate/ purificate of bacteriophages acting against *Staphylococcus aureus* and *Pseudomonas aeruginosa* at the titre of 10⁷ to 10¹¹ PFU/ml, 0.01 to 0.3 mg/ml of endolysin produced in *E*. coli with the sequence SEQ ID NO: 1, further 5 to 50 g of a filler and 4 to 13 g of a cryoprotectant per 100 ml of the mixture.

2. The mixture according to claim 1, **characterized in that** the bacteriophage is at least one phage from the group comprising the phage DSM 34647 and phage DSM 34648.

3. The mixture according to any of claims 1 or 2, **characterized in that** the filler is selected from the group comprising maltodextrin, fish gelatin and polyvinylpyrrolidone.

4. The mixture according to any of claims 1 to 3, **characterized in that** the cryoprotectant is mannitol.

5. An antimicrobial mixture comprising specific bacteriophages DSM 34647 and/or DSM 34648 with a phage titre of 10⁷ to 10¹¹ PFU/ml, 0.01 to 0.3 mg/ml of endolysin with the sequence SEQ ID NO: 1, further 5 to 50 g of a filler and 4 to 13 g of a cryoprotectant per 100 ml of the mixture for use in the treatment of bacterial infections caused by the pathogenic strains *Staphylococcus aureus* and *Pseudomonas aeruginosa* in the human as well as veterinary domain.

## Patentansprüche

1. Ein Gemisch zur Herstellung von Lyophilisaten mit antimikrobieller Wirkung gegen die Bakterien *Staphylococcus aureus* und *Pseudomonas aeruginosa,* das Bakteriophagen und lytische Enzyme mit optimaler Stabilität und Wirksamkeit der Wirkstoffe enthält, **dadurch gekennzeichnet, dass** sie ein Phagenlysat/Purifikat von Bakteriophagen enthält, die gegen *Staphylococcus aureus* und *Pseudomonas aeruginosa* wirken, mit einem Titer von 10⁷ bis 10¹¹ PFU/ml, 0,01 bis 0,3 mg/ml Endolysin, das in *E. coli* mit der Sequenz SEQ ID NO: 1 hergestellt wurde, sowie 5 bis 50 g Füllstoff und 4 bis 13 g Kryoprotektivum pro 100 ml der Mischung.

2. Das Gemisch nach Anspruch 1, **dadurch gekennzeichnet, dass** es sich bei dem Bakteriophagen um mindestens einen Phagen aus der Gruppe handelt, die den Phagen DSM 34647 und den Phagen DSM 34648 umfasst.

3. Das Gemisch nach einem der Ansprüche 1 oder 2, **dadurch gekennzeichnet, dass** der Füllstoff ausgewählt ist aus der Gruppe, die Maltodextrin, Fischgelatine und Polyvinylpyrrolidon umfasst.

4. Das Gemisch nach einem der Ansprüche 1 bis 3, **dadurch gekennzeichnet, dass** das Kryoprotektivum Mannitol ist.

5. Eine antimikrobielle Mischung, bestehend aus spezifischen Bakteriophagen DSM 34647 und/oder DSM 34648 mit einem Phagentiter von 10⁷ bis 10¹¹ PFU/ml, 0,01 bis 0,3 mg/ml Endolysin mit der Sequenz SEQ ID NO: 1, ferner 5 bis 50 g eines Füllstoffs und 4 bis 13 g eines Kryoprotektivums pro 100 ml der Mischung, zur Anwendung bei der Behandlung von bakteriellen Infektionen, die durch die pathogenen Stämme *Staphylococcus aureus* und *Pseudomonas aeruginosa* im Human- und Veterinärbereich verursacht werden.

## Revendications

1. Mélange pour la préparation de comprimés lyophilisés à effet antimicrobien contre les bactéries *Staphylococcus aureus* et *Pseudomonas aeruginosa,* contenant des bactériophages et des enzymes lytiques, avec une stabilité et une efficacité optimales des principes actifs, **caractérisé en ce qu'**il contient un lysat/ purificat de bactériophages agissant contre *Staphylococcus aureus* et *Pseudomonas aeruginosa* à un titre de 10⁷ à 10¹¹ PFU/ml, 0,01 à 0,3 mg/ml d' endolysine produite dans *E. coli* avec la séquence SEQ ID NO : 1, en outre 5 à 50 g d'une charge et 4 à 13 g d'un cryoprotecteur pour 100 ml du mélange.

2. Le mélange selon la revendication 1, **caractérisé en ce que** le bactériophage est au moins un phage du groupe comprenant le phage DSM 34647 et le phage DSM 34648.

3. Le mélange selon l'une quelconque des revendications 1 ou 2, **caractérisé en ce que** la charge est choisie dans le groupe comprenant la maltodextrine, la gélatine de poisson et la polyvinylpyrrolidone.

4. Le mélange selon l'une quelconque des revendications 1 à 3, **caractérisé en ce que** le cryoprotecteur est le mannitol.

5. Un mélange antimicrobien comprenant des bactériophages spécifiques DSM 34647 et/ou DSM 34648 avec un titre de phage de 10⁷ à 10¹¹ PFU/ml, 0,01 à 0,3 mg/ml d'endolysine avec la séquence SEQ ID NO : 1, en plus 5 à 50 g d'une charge et 4 à 13 g d'un cryoprotecteur pour 100 ml du mélange, destiné au traitement des infections bactériennes causées par les souches pathogènes *Staphylococcus aureus* et *Pseudomonas aeruginosa* dans le domaine humain et vétérinaire.
